# EUROPEAN PATENT APPLICATION

(11) **EP 1 974 725 A1**
(43) Date of publication of application: **01.10.2008**
(21) Application number: 06843208.7
(22) Date of filing: 26.12.2006
(51) Int. Cl.: A61K 9/22, A61K 31/222, A61K 47/32, A61K 47/38, A61P 13/02

(54) **SUSTAINED RELEASE PREPARATION AND METHOD FOR PRODUCTION THEREOF**

(30) Priority: 10.01.2006 JP 2006002164
(71) Applicant: Kissei Pharmaceutical Co., Ltd., Matsumoto-shi, Nagano 399-8710 (JP)
(72) Inventor: TAKEDA, Yasuhiro, Azumino-shi, Nagano 399-8304 (JP); WATANABE, Masayuki, Azumino-shi, Nagano 399-8304 (JP); NISHIDA, Ayumu, Azumino-shi, Nagano 399-8304 (JP)
(74) Representative: Hayes, Adrian Chetwynd
(86) International application number: PCT/JP2006/325824
(87) International publication number: WO 2007/080776

(57) **Abstract**

Disclosed is a sustained release preparation which comprises an active ingredient having a higher release rate at pH 4 compared to that in pH 1.2 or pH6.8 and exerts a controlled release of the active ingredient in a pH-independent manner. The sustained release preparation comprises ethyl (-)-2-[4-[2-[[(1S,2R)-2-hydroxy-2-(4-hydroxyphenyl)-1-methylethyl]amino]ethyl]-2,5-dimethyl-phenoxy]acetate hydrochloride as the active ingredient and a pH-independent gel-forming polymer and contains substantially no pH-controlling agent other than the polymer. The sustained release preparation can release the active ingredient in a pH-independent manner in the range from 1.2 to 6.8 and shows a constant release rate for a prolonged period of time. Therefore, the preparation is useful as a therapeutic agent for frequent urination/incontinence of urine which has a long-lasting effect.

## Description

### TECHNICAL FIELD

The present invention relates to a sustained release formulation of ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride that is useful as a therapeutic agent for pollakiuria and urinary incontinence, and a method for the production of the same.

### BACKGROUND ART

Sustained release formulations are more advantageous over immediate release formulations in terms of extended efficiencies of a drug by controlling the drug blood level, alleviation of the adverse effect, improvement of compliance through decreasing the frequency of administration and the like. Therefore, development of sustained release formulations of many drugs has been advanced in recent years. Variation of the pH in human gastrointestinal tract has been well known. For example, it has been reported that the pH is approximately 1 in the stomach; 5 to 6 in the duodenum; 6 to 7 in the jejunum; and approximately 8 in the ileum. Therefore, it has been sugguested that formulations which include a drug having pH-dependent solubilities or exhibiting a pH-dependent dissolution rate as an active ingredient are easily affected by alteration of the pH in the gastrointestinal tract, and the dissolution of such drug from the formulations is varied remarkably by the site in the gastrointestinal tract. Therefore, for the development of a sustained release formulation that includes a drug having pH-dependent solubilities or exhibiting a pH-dependent dissolution rate as an active ingredient, a sustained release formulation, the release of which is controlled in a pH-independent manner, is greatly desired.

Ethyl (-)-2-[4-[2-[[(1S,2R)-2-hydroxy-2-(4-hydroxyphenyl)-1-methylethyl]amino]ethyl]-2,5-dimethylphenoxy]-acetate hydrochloride represented by formula (I): 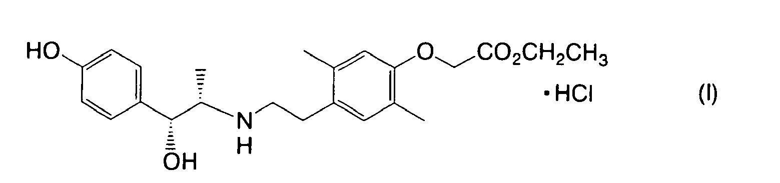 (hereinafter, referred to as compound (I)) has excellent β₃-adrenoceptor stimulating activities, and is useful as a therapeutic agent for pollakiuria and urinary incontinence. The compound (I) has a property to exhibit the highest dissolution rate at approximately pH 4 as compared with approximately pH 1.2 corresponding to the gastric juice and with approximately pH 6.8 corresponding to the intestinal juice. Accordingly, the compound (I) has the problem that the dissolution properties depend markedly on the pH alteration.

PH-independent gel-forming polymers such as hydroxypropyl cellulose have been widely used as a sustained release ingredient. However, it has been reported that in sustained release formulations using such a pH-independent gel-forming polymer, the pH in the swollen and gelated matrix in the gastrointestinal tract fundamentally varies depending on the pH in the circumstance, and accordingly, in cases where an active ingredient exhibits a pH-dependent dissolution rate, the dissolution of the active ingredient can not be controlled in a pH-independent manner (see nonpatent literature 1, patent literatures 1 and 2).

With respect to sustained release formulations of a drug exhibiting a pH-dependent dissolution rate, variety of methods for pH-independent controlled release have been conventionally reported in which an ingredient for regulating the solubility of a drug is added around the drug together with a sustained release ingredient. For example, in case of an acid addition salt of a basic drug such as compound (I), the solubility of the drug is generally high at pH in an acidic region, while the solubility is decreased as the pH is elevated. Accordingly, a process of adding an acidic ingredient (see patent literature 3), and a process of dispersing a drug in an enteric ingredient (see patent literature 4) have been proposed. Also, in case of an acidic drug, the solubility of the drug increases as the pH is elevated, and accordingly, addition of a basic ingredient is carried out (see patent literature 5). Furthermore, addition of a pH buffer agent to a matrix ingredient was also proposed (see patent literature 6) . Additionally, for drugs exhibiting a high dissolution rate of the drug at a pH around the intermediate of gastric juice and intestinal juice, a sustained release formulation comprising carboxyvinyl polymer or methylvinyl ether maleic anhydride copolymer as a polymer that causes swelling in a pH region higher than approximately pH 3 in combination with an enteric ingredient, was proposed (see patent literature 7).

Regarding pharmaceutical compositions containing compound (I) as an active ingredient, the following literatures have been known. WO2004/047830 discloses a film-coated tablet or capsule which comprises compound (I) and duloxetine hydrochloride as active ingredients (see patent literature 8) . WO2004/047838 discloses a formulation such as a film-coated tablet or a sugar coated tablet which comprises compound (I) and an antimuscarinic agent as active ingredients (see patent literature 9). Also, WO2005/042021 discloses a capsule which comrises compound (I) and tamsulosin hydrochloride as active ingredients (see patent literature 10). However, all of the formulations disclosed in these Patent literatures are directed to immediately releasing or enteric coated formulations, but any sustained release formulation, the release of which is controlled in a pH-independent manner was not described at all.
Nonpatent literature 1: Huang Y. B et al., "International Journal of Pharmaceutics", 2005, Vol. 289, P. 87-95
Patent literature 1: United States Patent No. 4792452
Patent literature 2: International Publication No. WO2003/000293 Pamphlet
Patent literature 3: Japanese laid-open Patent Publication No. Hei6-9388
Patent literature 4: Japanese laid-open Patent Publication No. Hei2-223533
Patent literature 5: Japanese laid-open Patent Publication No. Hei2-223533
Patent literature 6: Japanese laid-open Patent Publication No. Shou62-242615
Patent literature 7: Japanese laid-open Patent Publication No. Hei6-199657
Patent literature 8: International Publication No. WO2004/047830 Pamphlet
Patent literature 9: International Publication No. WO2004/047838 Pamphlet
Patent literature 10: International Publication No. WO2005/042021 Pamphlet

### DISCLOSURE OF THE INVENTION

An object of the present invention is to provide a sustained release formulation which comprises ethyl (-)-2-[4-[2-[[(1S,2R)-2-hydroxy-2-(4-hydroxyphenyl)-1-methyl-ethyl]amino]ethyl]-2,5-dimethylphenoxy]acetate hydrochloride as an active ingredient and exerts a controlled release of the active ingredient in a pH-independent manner, and a method for the production of the same.

The present inventors have examined the dissolution properties through adding a variety of solubility adjusting ingredients to the active ingredient, for the purpose of solving the aforementioned problem. Since acidic ingredients are often added in case of acid addition salts of a basic drug as described above, the present inventors firstly tried to add various acidic ingredients to compound (I), however, the pH dependent dissolution property was not improved at all. In addition, the present inventors tried to examine the dissolution property in which a basic ingredient or an enteric ingredient was added to compound (I), however, influence by the alteration of the pH could not be still improved sufficiently. Furthermore, in cases where the basic ingredient is added, decomposition of compound (I) itself was accelerated, and use of the basic ingredient was impractical.

Sustained release ingredients are broadly classified into water insoluble polymers and hydrophilic polymers. Further, water insoluble polymers and hydrophilic polymers are subclassified into pH-dependent polymers and pH-independent polymers, respectively. The present inventors have studied the dissolution properties of formulations containing any of these various sustained release ingredients. As a result, the pH-dependent polymers, and the water insoluble polymers could not improve the pH-dependent dissolution property. However, it was found surprisingly that pH-independent controlled releases are enabled by adding a hydrophilic polymer that forms a gel layer in an aqueous medium among the pH-independent polymers, without using other additive having a pH buffer capacity. Hitherto, when an active ingredient exhibits a pH-dependent dissolution rate, any sustained release formulation, the release of which is controlled in a pH-independent manner by using substantially a pH-independent gel-forming polymer alone, has not been known. Thus, it was wholly unexpected that a pH-independent gel-forming polymer exhibited excellent pH buffer effects for a drug such as compound (I) exhibiting a higher dissolution rate at pH 4 as compared with approximately pH 1.2 and approximately pH 6.8.

Accordingly, the present invention provides:
[1] a sustained release formulation which comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient, and a pH-independent gel-forming polymer;
[2] a sustained release formulation which comprises an active ingredient exhibiting a higher dissolution rate at pH 4 as compared with pH 1.2 or pH 6.8 and exerts a controlled release of the active ingredient in a pH-independent manner, wherein the sustained release formulation comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient, and a pH-independent gel-forming polymer;
[3] a sustained release formulation which comprises an active ingredient exhibiting a higher dissolution rate at pH 4 as compared with pH 1.2 or pH 6.8 and exerts a controlled release of said active ingredient in a pH-independent manner, wherein the sustained release formulation comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient, and a pH-independent gel-forming polymer, and contains substantially no pH-controlling agent other than said pH-independent gel-forming polymer;
[4] the sustained release formulation according to any one of [1] to [3], wherein the pH-independent gel-forming polymer is at least one selected from the group consisting of hydroxypropylmethyl cellulose, polyethylene oxide, polyvinyl alcohol, hydroxypropyl cellulose and methyl cellulose;
[5] the sustained release formulation according to [4], wherein the pH-independent gel-forming polymer is at least one selected from hydroxypropylmethyl cellulose, polyethylene oxide or polyvinyl alcohol;
[6] the sustained release formulation according to any one of [1] to [3], wherein the content of the pH-independent gel-forming polymer is 10% by weight or more with respect to the total weight of the formulation;
[7] the sustained release formulation according to any one of [1] to [3], wherein the content of the pH-independent gel-forming polymer is 10% by weight or more with respect to the active ingredient;
[8] the sustained release formulation according to [4] wherein the pH-independent gel-forming polymer is hydroxypropylmethyl cellulose;
[9] the sustained release formulation according to [8] which comprises hydroxypropylmethyl cellulose having a hydroxypropoxyl group content of 7 to 12% and a methoxy group content of 28 to 30%;
[10] the sustained release formulation according to [8] which comprises hydroxypropylmethyl cellulose having a hydroxypropoxyl group content of 4 to 12% and a methoxy group content of 19 to 24%;
[11] the sustained release formulation according to [8], wherein the content of hydroxypropylmethyl cellulose is 30% by weight or more with respect to the active ingredient;
[12] the sustained release formulation according to [8], wherein the content of hydroxypropylmethyl cellulose is 40% by weight or more with respect to the active ingredient;
[13] the sustained release formulation according to any one of [1] to [3] which further comprises a water insoluble material;
[14] the sustained release formulation according to [13], wherein the water insoluble material is at least one selected from higher alcohols, waxes, hardened oils, ethyl cellulose and ethyl acrylate methyl methacrylate copolymers and aminoalkyl methacrylate copolymer RS;
[15] the sustained release formulation according to [13] wherein the water insoluble material is at least one selected from higher alcohols and waxes;
[16] a method for pH-independent controlled release of a sustained release formulation which comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient, wherein the method comprises dispersing ethyl (-)-2-[4-[2-[[(1S,2R)-2-hydroxy-2-(4-hydroxyphenyl)-1-methylethyl]amino]ethyl)-2,5-dimethylphenoxy]acetate hydrochloride in a matrix of a pH-independent gel-forming polymer;
[17] a pH adjusting agent for use in a sustained release formulation which comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient and exerts a controlled release of the active ingredient in a pH-independent manner, wherein the agent consists of a pH-independent gel-forming polymer; and
[18] a method for preparing a sustained release formulation which comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient and exerts a controlled release of the active ingredient in a pH-independent manner, wherein the method comprises dispersing ethyl (-)-2-[4-[2-[[(1S,2R)-2-hydroxy-2-(4-hydroxyphenyl)-1-methylethyl)amino]ethyl)-2,5-dimethylphenoxy]acetate hydrochloride in a matrix of a pH-independent gel-forming polymer.

The term "sustained release formulation which exerts a controlled release in a pH-independent manner" as used herein refers to a sustained release formulation which exerts a controlled release in a pH-independent manner in the range of pH of 1.2 to 6.8, and more preferably, which exerts a controlled release in a pH-independent manner in any range of the dissolution rate of 20 to 40%, 40 to 60%, and 70% or greater when a dissolution test is carried out using test fluids at a pH 1.2, pH 4 and pH 6.8, at a paddle speed of 100 rpm according to Dissolution Test, method 2 (paddle method) in the Japanese Pharmacopoeia, fourteenth edition.

The term "controlled release in a pH-independent manner" as used herein refers to the condition where the difference of the dissolution rate in any of the range of 20 to 40%, 40 to 60%, and 70% or greater, i.e. the difference between the highest dissolution rate and the lowest dissolution rate among the range of 20 to 40%, 40 to 60%, and 70% or greater is preferably 20% or less, more preferably 15% or less, and most preferably 10% or less when a dissolution test is carried out using test fluids at a pH 1.2, pH 4 and pH 6.8, at a paddle speed of 100 rpm according to Dissolution Test, method 2 (paddle method) in the Japanese Pharmacopoeia, fourteenth edition.

PH-independent gel-forming polymers used in sustained release formulations of the present invention are a hydrophilic polymer which is swollen in an aqueous medium in a pH-independent manner to form a gel layer. Examples of such pH-independent gel-forming polymers include hydroxypropylmethyl cellulose, polyethylene oxide, polyvinyl alcohol, hydroxypropyl cellulose, methyl cellulose and the like.

Hydroxypropylmethyl cellulose (HPMC) is a cellulose derivative having a hydroxypropoxyl group and a methoxy group in the molecule, and various grades of hydroxypropylmethyl cellulose with varying hydroxypropoxyl group content and methoxy group content have been known.
Preferable hydroxypropylmethyl cellulose (HPMC) which may be used in sustained release formulations of the present invention includes HPMC with a hydroxypropoxyl group content in the range of 4 to 12% and a methoxy group content in the range of 19 to 30%. Specific examples of such HPMC include hydroxypropylmethyl cellulose 2910 with a hydroxypropoxyl group content of 7 to 12% and a methoxy group content of 28 to 30% (for example, Metolose 60SH type (Shin-Etsu Chemical Co., Ltd.). TC-5 type (Shin-Etsu Chemical Co. , Ltd.), METHOCEL E type (Dow Chemical) and the like); hydroxypropylmethyl cellulose 2906 with a hydroxypropoxyl group content of 4 to 7.5% and a methoxy group content of 27 to 30% (for example, Metolose 65SH type (Shin-Etsu Chemical Co., Ltd.), METHOCEL F type (Dow Chemical) and the like); and hydroxypropylmethyl cellulose 2208 with a hydroxypropoxyl group content of 4 to 12% and a methoxy group content of 19 to 24% (for example, Metolose 90SH type (Shin-Etsu Chemical Co., Ltd.), METHOCEL K type (Dow Chemical) and the like). Among these HPMC, HMPC with a hydroxypropoxyl group content of 7 to 12% and a methoxy group content of 28 to 30% (for example, Metolose 60SH type (Shin-Etsu Chemical Co., Ltd.), TC-5 type (Shin-Etsu Chemical Co., Ltd.), METHOCEL E type (Dow Chemical) and the like); and HMPC with a hydroxypropoxyl group content of 4 to 12% and a methoxy group content of 19 to 24% (for example, Metolose 90SH type (Shin-Etsu Chemical Co., Ltd.), METHOCEL K type (Dow Chemical) and the like) are suitable for high pH buffer effects; and HMPC with a hydroxypropoxyl group content of 7 to 12% and a methoxy group content of 28 to 30% (for example, Metolose 60SH type (Shin-Etsu Chemical Co. , Ltd.), TC-5 type (Shin-Etsu Chemical Co., Ltd.), METHOCEL E type (Dow Chemical) and the like) are most suitable for high pH buffer effects.
The viscosity of HPMC is not particularly limited, but it can be used to meet the intended dissolution profile through selecting the viscosity within the range of from 3 to 100000 mPa·s when measured at 20°C with a 2% aqueous solution, since dissolution of a drug is retarded more greatly as the viscosity of HPMC increases, in general. Among all, HPMC with a viscosity in the range of from 15 to 10000 mPa·s is suitable for sustained release effects.
These HPMC may be used in combination of two or more of them having different hydroxypropoxyl group content, methoxy group content and viscosity, as needed.

Polyethylene oxide (PEO) which may be used in sustained release formulations of the present invention includes PEO having a molecular weight of 100000 to 7000000. Specific examples of such PEO include PEO having a viscosity of 30 to 17600 mPa·s when measured at 25°C with a 5% aqueous solution (for example, POLYOX Water-Soluble Resin WSR N-10 NF for PEO with a viscosity of 30 to 50 mPa·s; POLYOX Water-Soluble Resin WSR N-80 NF for PEO with a viscosity of 55 to 90 mPa·s; POLYOX Water-Soluble Resin WSR N-750 NF for PEO with a viscosity of 600 to 1200 mPa·s; POLYOX Water-Soluble Resin WSR-205 NF for PEO with a viscosity of 4500 to 8800 mPa·s; POLYOX Water-Soluble Resin WSR-1105 NF for PEO with a viscosity of 8800 to 17600 mPa·s (Dow Chemical) and the like); PEO having a viscosity of 400 to 4000 mPa·s when measured at 25°C with a 2% aqueous solution (for example, POLYOX Water-Soluble Resin WSR N-12K NF for PEO with a viscosity of 400 to 800 mPa·s; POLYOX Water-Soluble Resin WSR N-60K NF for PEO with a viscosity of 2000 to 4000 mPa·s (Dow Chemical) and the like); PEO having a viscosity of 1650 to 10000 mPa·s when measured at 25°C with a 1% aqueous solution (for example, POLYOX Water Soluble Resin WSR-301 NF for PEO with a viscosity of 1650 to 5500 mPa·s; POLYOX Water-Soluble Resin WSR Coagulant NF for PEO with a viscosity of 5500 to 7500 mPa·s; POLYOX Water-Soluble Resin WSR-303 NF for PEO with a viscosity of 7500 to 10000 mPa·s (Dow Chemical) and the like), and the like.
More preferably, PEO having a molecular weight of 100000 to 5000000, and having a viscosity of 30 to 17600 mPa·s when measured at 25°C with a 5% aqueous solution; a viscosity of 400 to 4000 mPa·s when measured at 25°C with a 2% aqueous solution; and a viscosity of 1650 to 7500 mPa·s when measured at 25°C with a 1% aqueous solution, is suitable for pH buffer effects and sustained release effects.

Polyvinyl alcohol (PVA) which may be used in sustained release formulations of the present invention includes PVA having a saponification degree of 78.0 mol% to 96.0 mol% and a viscosity of 2 to 100 mPa·s when measured at 20°C with a 4% aqueous solution. More specifically, PVA having a saponification degree of 86.5 mol% to 89.0 mol%, and a viscosity of 4.8 to 46.0 mPa·s when measured at 20°C with a 4% aqueous solution (for example, EG-05, EL-5 for PVA with a viscosity of 4.8 to 5.8 mPa·s; EG-25 for PVA with a viscosity of 20.5 to 24.5 mPa·s; EG-30, GH-17 for PVA with a viscosity of 27.0 to 33.0 mPa·s; EG-40 for PVA with a viscosity of 40.0 to 46.0 mPa·s (Nippon Synthetic Chemical Industry Co., Ltd.) and the like) is suitable for pH buffer effects and sustained release effects.

The content of these pH-independent gel-forming polymers may vary depending on the type of the polymers, but the pH-independent gel-forming polymers are usually added in an amount of 10% by weight or more with respect to the active ingredient for achieving pH buffer effects. The pH-independent gel-forming polymers are usually blended in an amount of 10% by weight or more with respect to the total weight of the formulation. More specifically, when hydroxypropylmethyl cellulose is used, the content of hydroxypropylmethyl cellulose is preferably 30% by weight or more with respect to the active ingredient, and more preferably 40% by weight or more with respect to the active ingredient. When polyethylene oxide is used, the content of polyethylene oxide is preferably 10% by weight or more with respect to the active ingredient, and more preferably 15% by weight or more with respect to the active ingredient. When polyvinyl alcohol is used, the content of polyvinyl alcohol is preferably 35% by weight or more with respect to the active ingredient, and more preferably 40% by weight or more with respect to the active ingredient.

Among these pH-independent gel-forming polymers, hydroxypropylmethyl cellulose, polyethylene oxide or polyvinyl alcohol is preferable, and hydroxypropylmethyl cellulose or polyethylene oxide is more preferable.

Also, these pH-independent gel-forming polymers may be used in combination of two or more thereof as needed, and the dissolution rate of compound (I) from the matrix can be arbitrarily regulated by altering the type, viscosity and amount of addition of the pH-independent gel-forming polymer.

Sustained release formulations of the present invention contain substantially no pH-controlling agent other than pH-independent gel-forming polymers since pH-controlling agents such as acidic ingredients, basic ingredients, enteric ingredients, pH-dependent polymers and the like other than pH-independent gel-forming polymers detract from the effect of pH-independently controlled release achieved by the present invention. The term "substantially" as used herein means that pH-controlling agents other than pH-independent gel-forming polymers are permissible unless its content is large enough to antagonize the effect of the present invention.

Furthermore, in sustained release formulations of the present invention, pH-independent gel-forming polymers may be used in combination with other sustained release ingredients such that the pH-independent controlled release may not be deteriorated. Examples of such sustained release ingredients include pH-dependent gel-forming polymers, water insoluble materials and the like.

Specific examples of pH-dependent gel-forming polymers which may be used in sustained release formulations of the present invention include carboxyvinyl polymers, sodium alginate and the like. These pH-dependent gel-forming polymers may be used alone, or in combination of two or more thereof.

The water insoluble materials which may be used in sustained release formulations of the present invention are not particularly limited as long as they have not pH-buffering abilities and can modify, more preferably retard the dissolution rate of the active ingredient. Examples of such water insoluble materials include higher alcohols such as myristyl alcohol, cetyl alcohol, stearyl alcohol and the like; waxes such as carnauba wax, yellow beeswax, lanolin, microcrystalline wax and the like; hardened oils such as castor oil, cottonseed oil, soybean oil, rapeseed oil, beef tallow oil and the like; ethyl cellulose; methacrylic acid copolymers such as aminoalkyl methacrylate copolymer RS, ethyl acrylate methyl methacrylate copolymers and the like. More specifically, higher alcohols, waxes or ethyl cellulose are preferable. Among them, stearyl alcohol, carnauba wax or ethyl cellulose is more preferable. These water insoluble materials may be used alone, or two or more thereof may be used in combination. A use of such a water insoluble material in combination with a pH-independent gel-forming polymer enables still further sustained release effects. In addition, the dissolution rate of compound (I) from the matrix can be regulated arbitrarily by changing the ratio of the water insoluble material and the pH-independent gel-forming polymer.

Furthermore, sustained release formulations of the present invention may include a variety of additives as used in the manufacture of formulations other than the sustained release ingredients as described above as long as they are not disadvantageous to the effect of the present invention. Examples of such additives include fillers, binders, disintegrants, lubricants, plasticizer, coating agents, buffers, colorants and the like.

Examples of fillers include lactose, crystalline cellulose, mannitol, starch and the like. Examples of binders include hydroxypropyl cellulose, hydroxypropylmethyl cellulose, gelatin, gum arabic, sodium alginate, polyethylene glycol, stearyl alcohol, carnauba wax and the like. Examples of disintegrants include low-substituted hydroxypropyl cellulose, croscarmellose sodium, carmellose calcium, crospovidone, carboxymethyl starch sodium and the like. Examples of lubricants include magnesium stearate, stearic acid, calcium stearate, talc, sodium stearyl fumarate and the like.

Dosage forms of sustained release formulations according to the present invention include a variety of dosage forms such as powders, granules, tablets and capsules. Among them, tablet is most preferable but not limited thereto.

Next, methods for preparing sustained release formulations of the present invention will be explained.
In sustained release formulations of the present invention, compound (I) is dispersed in a matrix of pH-independent gel-forming polymers, and methods for preparing the same are not particularly limited, but sustained release formulations of the present invention are preferably prepared according to the methods as described below.

Compound (I), a pH-independent gel-forming polymer, and other additive as needed are mixed. Then, the resulting mixture is granulated using an appropriate binder according to a conventional granulating process such as a high-shear granulation process, a fluidized bed granulation process, or a tumbling granulation process, followed by drying and sizing to prepare granules.

Alternatively, compound (I) and other additive as needed are mixed, and the resulting mixure is granulated using an appropriate binder according to a conventional granulating process such as a high-shear granulation process, a fluidized bed granulation process, or a tumbling granulation process. Thereafter, drying and sizing are carried out, and a pH-independent gel-forming polymer is mixed therewith to prepare granules.

As the binders which may be used in the granulation described above, an organic solvent, e.g., an alcohol such as methanol or ethanol, acetone, methylene chloride or the like; or water is used in the form of a binder liquid. Alternatively, a solution or a suspension prepared by dissolving or dispersing a binder in such an organic solvent or water is suitable for achieving a favorable pH-buffering effect.

Alternatively, in case of preparing according to a dry granulation process, a conventional dry granulation apparatus is used to form agglomerates through pressurizing the mixture of compound (I), a pH-independent gel-forming polymer and other additives as needed, followed by sizing to prepare granules.

Moreover, in case of compositions which contains an ingredient having a low melting point such as a higher alcohol, a wax, polyethylene glycol and the like, compound (I), a pH-independent gel-forming polymer, the ingredient having alow melting point, and other additives as needed are mixed. Thereafter, the resulting mixture is granulated by a melt granulation process, followed by sizing to prepare granules.

To the granules prepared according to the processes as described above is added a lubricant as needed, followed by tableting with a tableting machine to provide a tablet. When a lubricant is added, the granules and the lubricant may be mixed with a conventional mixing process using any of various powder mixing machines, and the mixture may be subjected to tableting. Alternatively, tableting with an external lubricating method can achieve a favorable pH-buffering effect.

Sustained release formulations of the present invention prepared as described above exhibit a controlled release in a pH-independent manner, and have a constant dissolution rate. Therefore, sustained release formulations of the present invention are extremely useful as a therapeutic drug having an extended effect for diseases such as pollakiuria and urinary incontinence.

Sustained release formulations of the present invention accomplish a pH-independent dissolution in the range of pH 1.2 to 6.8, and exhibit a constant dissolution rate for a long period of time. Therefore, sustained release formulations of the present invention exhibit a constant dissolution rate without being affected by the pH environment in the gastrointestinal tract, and can exert an excellent extended effect with less variation in the individual or among the individuals. Moreover, sustained release formulations of the present invention can be prepared by using a pH-independent gel-forming polymer without adding other additive having a pH buffer effect, and accordingly can be readily prepared and are suitable for an industrial production.

### Brief Description of the Drawings

[Fig. 1] Fig. 1 shows the dissolution rate of compound (I) from a tablet including compound (I) and lactose. The ordinate shows the dissolution rate (%), and the abscissa shows the dissolution time (min).
[Fig. 2] Fig. 2 shows the dissolution rate of compound (I) from a tablet prepared by adding ethyl cellulose as a pH-independent water insoluble polymer to compound (I) and lactose. The ordinate shows the dissolution rate (%), and the abscissa shows the dissolution time (min).
[Fig. 3] Fig. 3 shows the dissolution rate of compound (I) from a tablet prepared by adding succinic acid as an acidic material to compound (I) and lactose. The ordinate shows the dissolution rate (%), and the abscissa shows the dissolution time (min).
[Fig. 4] Fig. 4 shows the dissolution rate of compound (I) from a tablet prepared by adding dipotassium hydrogenphosphate as a basic material to compound (I) and lactose. The ordinate shows the dissolution rate (%), and the abscissa shows the dissolution time (min).
[Fig. 5] Fig. 5 shows the dissolution rate of compound (I) from a tablet prepared by adding methacrylic acid copolymer (Eudragit S) as an enteric polymer to compound (I) and lactose. The ordinate shows the dissolution rate (%), and the abscissa shows the dissolution time (min).
[Fig. 6] Fig. 6 shows the dissolution rate of compound (I) from a tablet prepared by adding carboxyvinyl polymer as a pH-dependent gel-forming polymer to compound (I) and lactose. The ordinate shows the dissolution rate (%), and the abscissa shows the dissolution time (min).
[Fig. 7] Fig. 7 shows the dissolution rate of compound (I) from a tablet prepared by adding hydroxypropylmethyl cellulose 2910 as a pH-independent gel-forming polymer to compound (I) and lactose. The ordinate shows the dissolution rate (%), and the abscissa shows the dissolution time (min).
[Fig. 8] Fig. 8 shows the dissolution rate of compound (I) from a tablet prepared by adding polyethylene oxide as a pH-independent gel-forming polymer to compound (I) and lactose. The ordinate shows the dissolution rate (%), and the abscissa shows the dissolution time (min).
[Fig. 9] Fig. 9 shows the dissolution rate of compound (I) from a tablet prepared by adding hydroxypropylmethyl cellulose 2910 as a pH-independent gel-forming polymer and stearyl alcohol as a water insoluble material to compound (I) and lactose. The ordinate shows the dissolution rate (%), and the abscissa shows the dissolution time (min).
[Fig. 10] Fig. 10 shows the dissolution rate of compound (I) from a tablet prepared by a melt granulation process with a mixture of compound (I), hydroxypropylmethyl cellulose 2910 as a pH-independent gel-forming polymer and polyethylene glycol. The ordinate shows the dissolution rate (%), and the abscissa shows the dissolution time (min).
[Fig. 11] Fig. 11 shows the dissolution rate of compound (I) from a tablet prepared by granulation of a mixture of compound (I) and hydroxypropylmethyl cellulose 2910 as a pH-independent gel-forming polymer using a hydroxypropyl cellulose solution in ethanol. The ordinate shows the dissolution rate (%), and the abscissa shows the dissolution time (min).
[Fig. 12] Fig. 12 shows the dissolution rate of compound (I) from a tablet prepared by granulating a mixture of compound (I) and lactose using an aqueous solution of hydroxypropyl cellulose, followed by adding hydroxypropylmethyl cellulose 2910 as a pH-independent gel-forming polymer. The ordinate shows the dissolution rate (%), and the abscissa shows the dissolution time (min).
[Fig. 13] Fig. 13 shows the dissolution rate of compound (I) from a tablet prepared by adding polyvinyl alcohol as a pH-independent gel-forming polymer to compound (I). The ordinate shows the dissolution rate (%), and the abscissa shows the dissolution time (min).
[Fig. 14] Fig. 14 shows the dissolution rate of compound (I) from a tablet prepared by mixing compound (I) with hydroxypropylmethyl cellulose 2910 as a pH-independent gel-forming polymer, followed by adding magnesium stearate. The ordinate shows the dissolution rate (%), and the abscissa shows the dissolution time (min).
[Fig. 15] Fig. 15 shows the dissolution rate of compound (I) from a tablet prepared by mixing hydroxypropylmethyl cellulose 2910 as a pH-independent gel-forming polymer with compound (I), followed by tableting using a die and punches precoated with magnesium stearate. The ordinate shows the dissolution rate (%), and the abscissa shows the dissolution time (min).
[Fig. 16] Fig. 16 shows the dissolution rate of compound (I) from a tablet prepared by adding hydroxypropylmethyl cellulose 2208 as a pH-independent gel-forming polymer to compound (I) and lactose. The ordinate shows the dissolution rate (%), and the abscissa shows the dissolution time (min).

### Best Mode for Carrying Out the Invention

Aspects of the present invention will be explained in more detail by way of the following Examples, Comparative Examples and Test Example, however, the invention is not limited thereto.

### [Examples]

### Test Example 1

### Dissolution test

Using the tablets prepared in the following Comparative Examples 1 to 6 and Examples 1 to 5, dissolution rate of compound (I) from these tablets was examined. Evaluation of the dissolution rate was carried out using 900 ml of 1st fluid (pH 1.2) and 2nd fluid (pH 6.8) defined in Disintegration Test in the Japanese Pharmacopoeia, fourteenth edition, and McIlvaine buffer (pH 4.0), at a paddle speed of 100 rounds per minute (rpm) according to Dissolution Test, method 2 (paddle method) in the Japanese Pharmacopoeia, fourteenth edition. After initiating the dissolution test, sampling was conducted with time, and the absorbance of the samples was measured using an absorption spectrometer (wavelength: 225 nm) to determine the dissolution rate of compound (I).

### Comparative Example 1

| Compound (I) | 50 mg |
|---|---|
| Lactose | 100 mg |
| | (Total: 150 mg/tablet) |

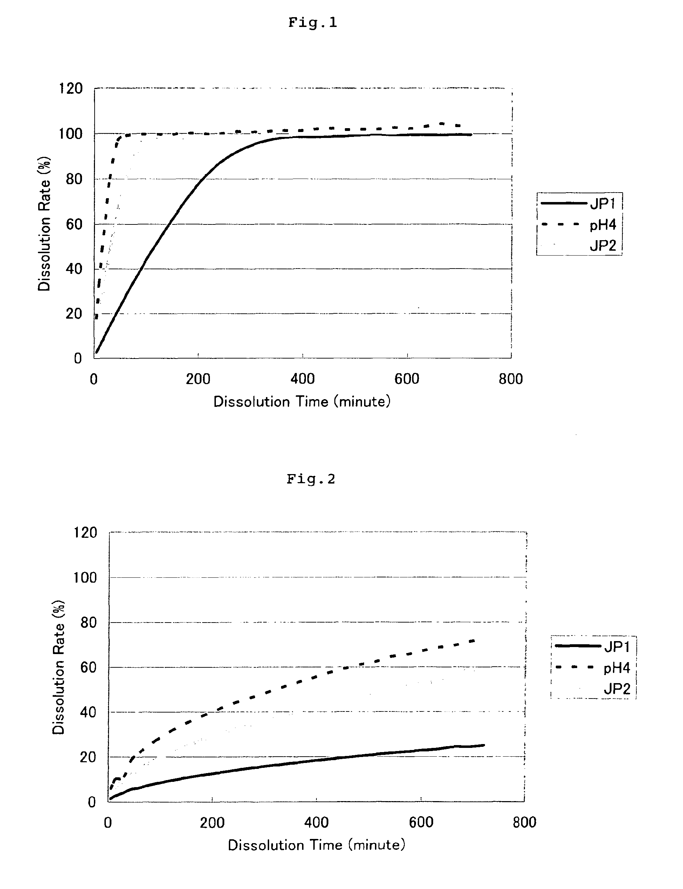

500 mg of compound (I) and 1000 mg of lactose were mixed in a mortar, and the resulting powder was compression-molded with a single punch tableting machine to prepare tablets having a weight of 150 mg each. The results of a dissolution test are shown in Fig. 1.
As a result, the formulation of Reference Example 1 exhibited dissolution properties where the dissolution rate was highest with pH 4 test fluid, while the dissolution rate was slightly lower with 2nd fluid, and was markedly lower with 1st fluid.

### Comparative Example 2

| Compound (I) | 50 mg |
|---|---|
| Lactose | 70 mg |
| Ethyl cellulose | 30 mg |
| | (Total 150 mg/tablet) |

According to the composition described above, 500 mg of compound (I), 700 mg of lactose and 300 mg of ethyl cellulose were mixed in a mortar, and the resulting powder was compression-molded with a single punch tableting machine to prepare tablets having a weight of 150 mg each. The results of a dissolution test are shown in Fig. 2.
As a result, the formulation of Comparative Example 2, which contained ethyl cellulose that is a pH-independent water insoluble polymer and does not form a gel layer, exhibited a merely retarded dissolution rate as compared with those of Comparative Example 1, with no improvement in the pH-dependence of the dissolution rate.

### Comparative Example 3

| Compound (I) | 50 mg |
|---|---|
| Lactose | 50 mg |
| Succinic acid | 50 mg |
| | (Total 150 mg/tablet) |

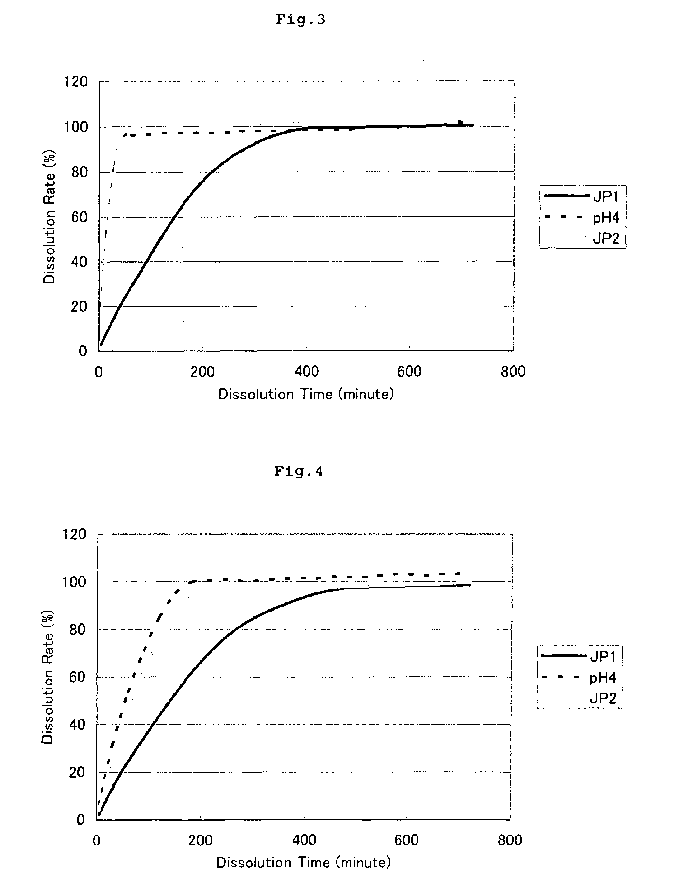

According to the composition described above, 500 mg of compound (I), 500 mg of lactose and 500 mg of succinic acid were mixed in a mortar, and the resulting powder was compression-molded with a single punch tableting machine to prepare tablets having a weight of 150 mg each. The results of a dissolution test are shown in Fig. 3.
As a result, in the formulation of Reference Example 3 which contained succinic acid as an acidic material, the pH dependence between pH 4 test fluid and 2nd fluid was improved, while the dissolution rate of 1st fluid was markedly lower as compared with those of pH 4 test fluid and 2nd fluid, suggesting that the pH-dependence of the dissolution rate was not completely improved.

### Comparative Example 4

| Compound (I) | 50 mg |
|---|---|
| Lactose | 50 mg |
| Dipotassium hydrogenphosphate | 50 mg |
| | (Total 150 mg/tablet) |

According to the composition described above, 500 mg of the compound (I), 500 mg of lactose and 500 mg of dipotassium hydrogenphosphate were mixed in a mortar, and the resulting powder was compression-molded with a single punch tableting machine to prepare tablets having a weight of 150 mg each. The results of a dissolution test are shown in Fig. 4.
As a result, in the formulation of Comparative Example 4 which contained dipotassium hydrogenphosphate as a basic material, the pH dependence between pH 4 test fluid and 2nd fluid was improved, while the dissolution rate of 1st fluid was markedly lower as compared with those of pH 4 test fluid and 2nd fluid, suggesting that the pH-dependence of the dissolution rate was not completely improved.

### Comparative Example 5

| Compound (I) | 50 mg |
|---|---|
| Lactose | 70 mg |
| Methacrylic acid copolymer | 30 mg |
| | (Total 150 mg/tablet) |

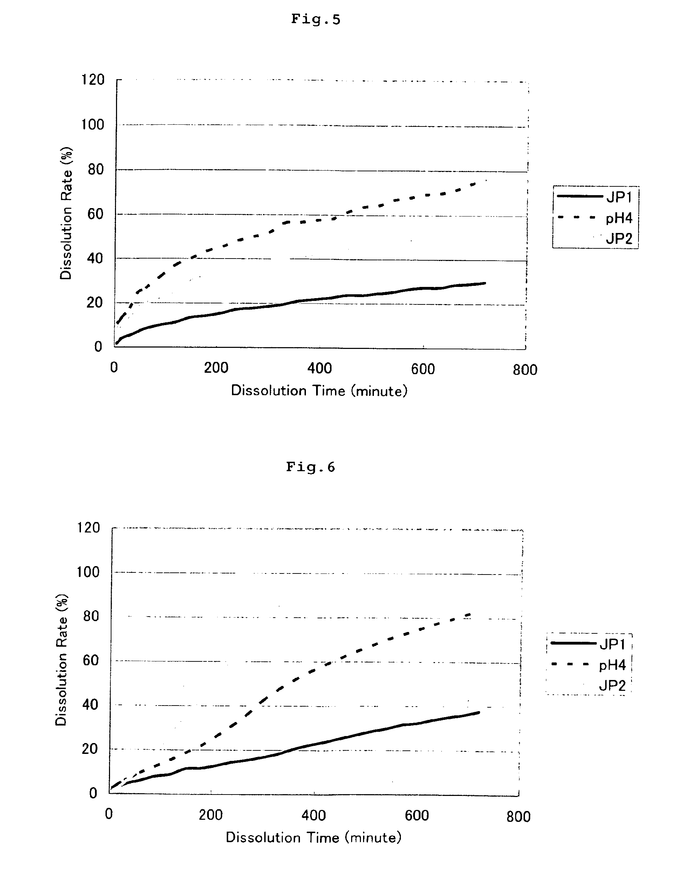

According to the composition described above, 500 mg of compound (I), 700 mg of lactose and 300 mg of methacrylic acid copolymer (Eudragit S, Degussa) as an enteric ingredient were mixed in a mortar, and the resulting powder was compression-molded with a single punch tableting machine to prepare tablets having a weight of 150 mg each. The results of a dissolution test are shown in Fig. 5.
As a result, the formulation of Comparative Example 5 which contained Eudragit S as an enteric polymer exhibited a merely retarded dissolution rate as compared with those of Comparative Example 1, with no improvement in the pH-dependence of the dissolution rate.

### Comparative Example 6

| Compound (I) | 50 mg |
|---|---|
| Lactose | 70 mg |
| Carboxyvinyl polymer | 30 mg |
| | (Total 150 mg/tablet) |

According to the composition described above, 500 mg of compound (I), 700 mg of lactose and 300 mg of carboxyvinyl polymer (Carbopol 974P NF, Noveon) were mixed in a mortar, and the resulting powder was compression-molded with a single punch tableting machine to prepare tablets having a weight of 150 mg each. The results of a dissolution test are shown in Fig. 6.
As a result, the formulation of Comparative Example 6 which contained carboxyvinyl polymer as a pH-dependent gel-forming polymer exhibited a merely retarded dissolution rate as compared with those of Comparative Example 1, with no improvement in the pH-dependence of the dissolution rate.

### Example 1

| Compound (I) | 50 mg |
|---|---|
| Lactose | 50 mg |
| Hydroxypropylmethyl cellulose 2910 | 50 mg |
| | (Total 150 mg/tablet) |

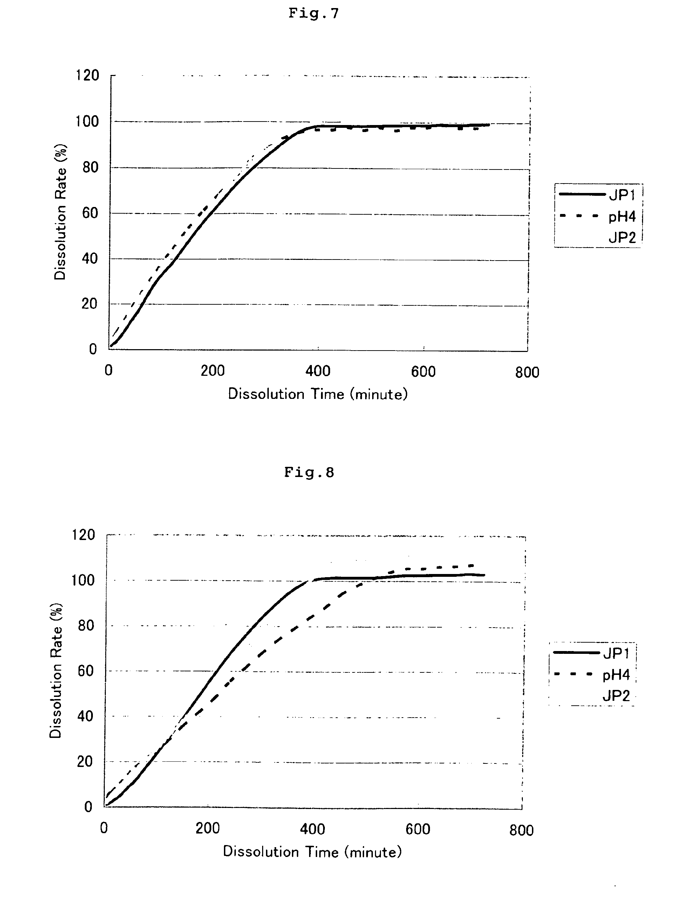

According to the composition described above, 500 mg of compound (I), 500 mg of lactose and 500 mg of hydroxypropylmethyl cellulose 2910 (Metolose 60SH50, Shin-Etsu Chemical Co., Ltd.) were mixed in a mortar, and the resulting powder was compression-molded with a single punch tableting machine to prepare tablets having a weight of 150 mg each. The results of a dissolution test are shown in Fig. 7.
Consequently, the formulation of Example 1 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Example 2

| Compound (I) | 50 mg |
|---|---|
| Lactose | 50 mg |
| Polyethylene oxide | 50 mg |
| | (Total 150 mg/tablet) |

According to the composition described above, 500 mg of compound (I), 500 mg of lactose and 500 mg of polyethylene oxide (POLYOX WSR 301, Dow Chemical) were mixed in a mortar, and the resulting powder was compression-molded with a single punch tableting machine to prepare tablets having a weight of 150 mg each. The results of a dissolution test are shown in Fig. 8.
Consequently, the formulation of Example 2 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Example 3

| Compound (I) | 50 mg |
|---|---|
| Lactose | 50 mg |
| Hydroxypropylmethyl cellulose 2910 | 25 mg |
| Stearyl alcohol | 25 mg |
| | (Total 150 mg/tablet) |

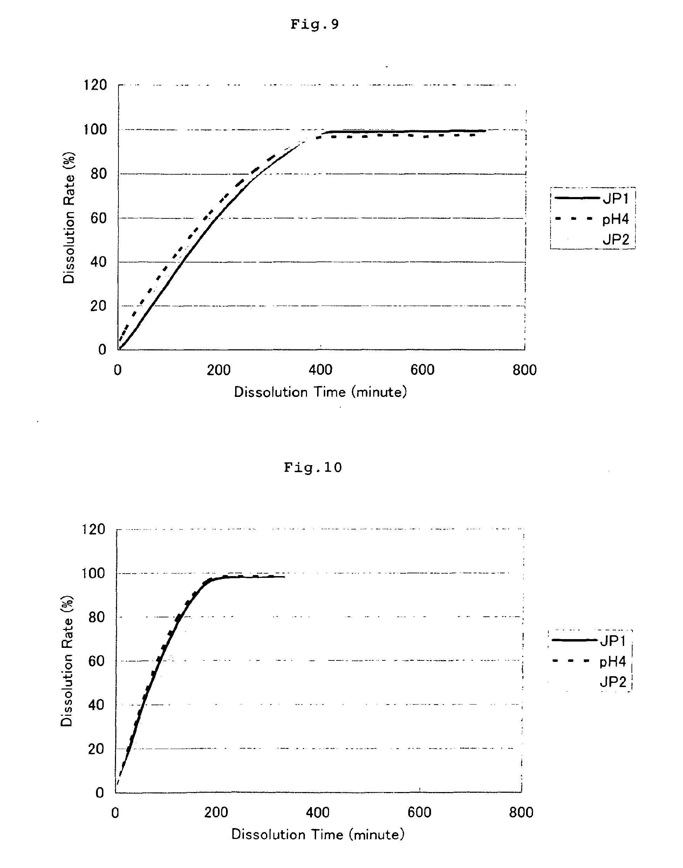

According to the composition described above, 500 mg of compound (I), 500 mg of lactose, 250 mg of hydroxypropylmethyl cellulose 2910 (Metolose 60SH50, Shin-Etsu Chemical Co. , Ltd.) and 250 mg of stearyl alcohol were mixed in a mortar. The resulting powder was placed in a test tube, and subjected to melt granulation in a water bath heated at about 75°C. The granular materials were pulverized and sized in a mortar, then passed through a 50 mesh sieve. The resulting granules were compression-molded with a single punch tableting machine to prepare tablets having a weight of 150 mg each. The results of a dissolution test are shown in Fig. 9.
Consequently, the formulation of Example 3 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Example 4

| Compound (I) | 160 mg |
|---|---|
| Hydroxypropylmethyl cellulose 2910 | 260 mg |
| Polyethylene glycol 6000 | 105 mg |
| Magnesium stearate | 0.53 mg |
| | (Total 525.53 mg/tablet) |

According to the composition described above, 320 g of compound (I), 520 g of hydroxypropylmethyl cellulose 2910 (TC-5R, Shin-Etsu Chemical Co., Ltd.) and 210 g of polyethylene glycol 6000 (Sanyo Chemical Industries, Ltd.) were mixed in a heated high shear granulator (VG-10, Powrex) and subjected to melt granulation by polyethylene glycol 6000. The granular materials were sized with a power mill (P-02S, Dalton). The resulting powder was lubricated with a V-blender after adding 1.06 g of magnesium stearate (TAIHEI CHEMICAL INDUSTRIAL CO. , LTD.). Then the resulting lubricated powder was compression-molded with a rotary tableting machine to prepare tablets having a weight of 525.53 mg each. The results of a dissolution test are shown in Fig. 10.
Consequently, the formulation of Example 4 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Example 5

| Compound (I) | 160 mg |
|---|---|
| Hydroxypropylmethyl cellulose 2910 | 240 mg |
| Hydroxypropyl cellulose | 16 mg |
| Magnesium stearate | 2.08 mg |
| | (Total 418.08 mg/tablet) |

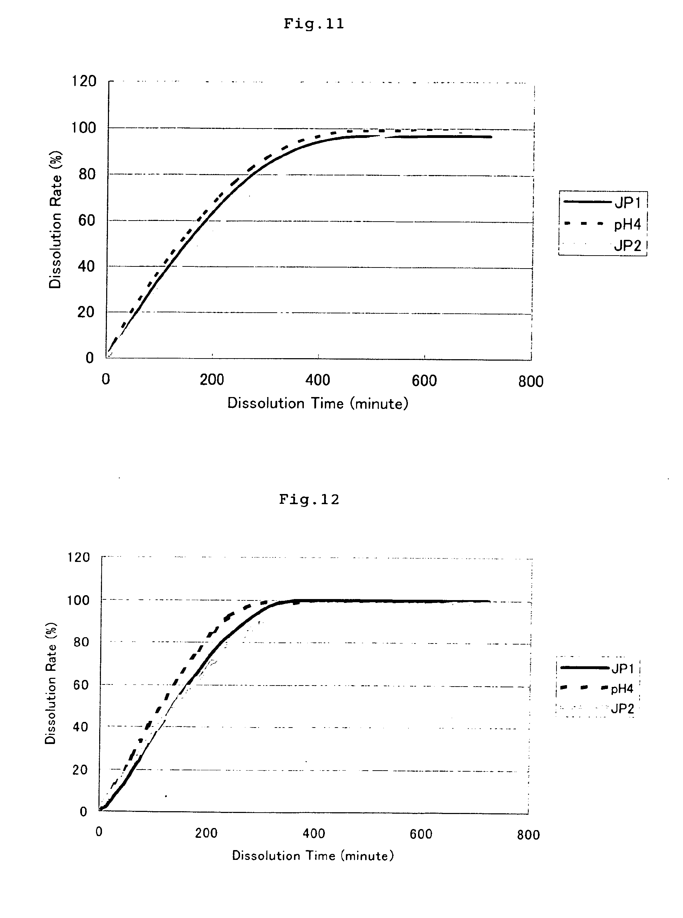

According to the composition described above, 320 g of compound (I) and 480 g of hydroxypropylmethyl cellulose 2910 (TC-5S, Shin-Etsu Chemical Co. , Ltd.) were mixed in a high shear granulator (VG-10, Powrex), and 640 g of a 5% solution of hydroxypropyl cellulose (HPC-L, NIPPON SODA CO., LTD.) in ethanol was added thereto to granulate. The resulting granular materials were dried and then sized with a power mill (P-02S, Dalton) . The resulting powder was lubricated with a V-blender after adding 4.16 g of magnesium stearate (TAIHEI CHEMICAL INDUSTRIAL CO., LTD.). The resulting lubricated powder was compression-molded with a rotary tableting machine to prepare tablets having a weight of 418.08 mg each. The results of a dissolution test are shown in Fig. 11.
Consequently, the formulation of Example 5 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Example 6

| Compound (I) | 50 mg |
|---|---|
| Lactose | 50 mg |
| Hydroxypropylmethyl cellulose 2910 | 50 mg |
| Hydroxypropyl cellulose | 1.25 mg |
| | (Total 151.25 mg/tablet) |

According to the composition described above, 1000 mg of compound (I) and 1000 mg of lactose were mixed in a mortar, and 500 mg of a 5% aqueous solution of hydroxypropyl cellulose (HPC-L, NIPPON SODA CO. , LTD.) was added thereto to granulate. The resulting granular materials were dried and mixed with 1000 mg of hydroxypropylmethyl cellulose 2910 (Metolose 60SH50, Shin-Etsu Chemical Co. , Ltd.) in a mortar. The resulting powder was compression-molded with a single punch tableting machine to prepare tablets having a weight of 151.25 mg each. The results of a dissolution test are shown in Fig. 12.
Consequently, the formulation of Example 6 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Example 7

| Compound (I) | 50 mg |
|---|---|
| Polyvinyl Alcohol | 100 mg |
| | (Total 150 mg/tablet) |

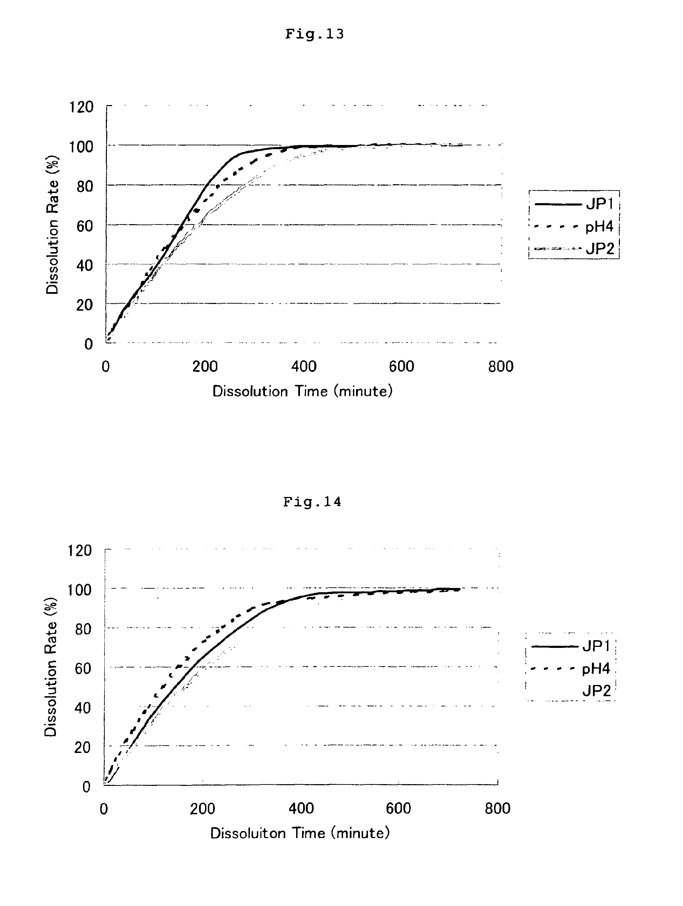

According to the composition described above, 500 mg of compound (I) and 1000 mg of polyvinyl alcohol (GH-17, The Nippon Synthetic Chemical Industry Co., Ltd.) were mixed in a mortar, and the resulting powder was compression-molded with a single punch tableting machine to prepare tablets having a weight of 150 mg each. The results of a dissolution test are shown in Fig. 13.
Consequently, the formulation of Example 7 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Example 8

| Compound (I) | 50 mg |
|---|---|
| Hydroxypropylmethyl cellulose 2910 | 100 mg |
| Magnesium stearate | 1.5 mg |
| | (Total 151.5 mg/tablet) |

According to the composition described above, 500 mg of compound (I) and 1000 mg of hydroxypropylmethyl cellulose 2910 (TC-5S, Shin-Etsu Chemical Co., Ltd.) were mixed in a mortar. After adding 15 mg of magnesium stearate (TAIHEI CHEMICAL INDUSTRIAL CO., LTD.), the resulting powder was lubricated in a plastic bag. The resulting lubricated powder was compression-molded with a single punch tableting machine to prepare tablets having a weight of 151.5 mg each. The results of a dissolution test are shown in Fig. 14.
Consequently, the formulation of Example 8 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Example 9

| Compound (I) | 50 mg |
|---|---|
| Hydroxypropylmethyl cellulose 2910 | 100 mg |
| Magnesium stearate | a trace amount |
| | (Total 150 mg/tablet) |

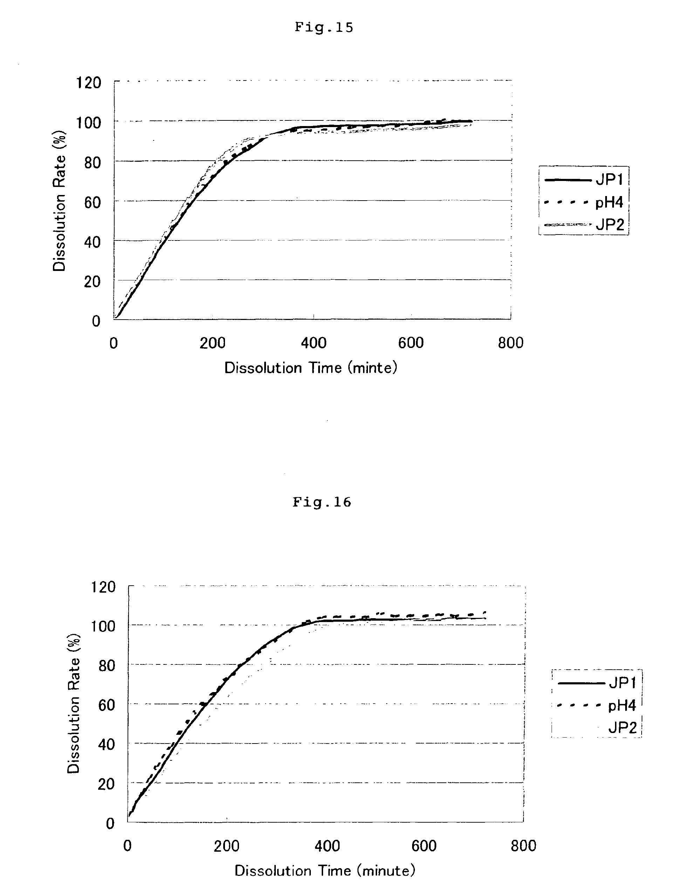

According to the composition described above, 500 mg of compound (I) and 1000 mg of hydroxypropylmethyl cellulose 2910 (TC-5S, Shin-Etsu Chemical Co., Ltd.) were mixed in a mortar. A die and punches of a single punch tableting machine were pre coated with magnesium stearate (TAIHEI CHEMICAL INDUSTRIAL CO., LTD.) and excessive lubricant was removed by compressed air, and then the resulting mixed powder was compression-molded to prepare tablets having a weight of 150 mg each. The results of a dissolution test are shown in Fig. 15.
Consequently, the formulation of Example 9 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties. Furthermore, the dissolution profile of the formulation of Example 9 with external lubrication had better pH-independent dissolution properties than the formulation of Example 8 with normal lubrication.

### Example 10

| Compound (I) | 50 mg |
|---|---|
| Lactose | 50 mg |
| Hydroxypropylmethyl cellulose 2208 | 50 mg |
| | (Total 150 mg/tablet) |

According to the composition described above, 500 mg of compound (I), 500 mg of lactose and 500 mg of hydroxypropylmethyl cellulose 2208 (Metolose 90SH100SR, Shin-Etsu Chemical Co., Ltd.) were mixed in a mortar, and the resulting powder was compression-molded with a single punch tableting machine to prepare tablets having a weight of 150 mg each. The results of a dissolution test are shown in Fig. 16.
Consequently, the formulation of Example 10 gave the result that the pH dependence of the dissolution rate among 1st fluid, pH 4 test fluid and 2nd fluid was markedly improved in any of the range of the dissolution rate being 20 to 40%, 40 to 60%, and 70% or greater, suggesting pH-independent dissolution properties.

### Industrial Applicability

The sustained release formulations of the present invention exhibit a pH-independent dissolution property in the range of pH 1.2 to 6.8. Moreover, the sustained release formulations of the present invention exhibit a constant dissolution rate for a long period of time. Accordingly, the sustained release formulations of the present invention are useful as a therapeutic agent with an extended effect for pollakiuria and urinary incontinence.

## Claims

1. A sustained release formulation which comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient, and a pH-independent gel-forming polymer.

2. A sustained release formulation which comprises an active ingredient exhibiting a higher dissolution rate at pH 4 as compared with pH 1.2 or pH 6.8 and exerts a controlled release of said active ingredient in a pH-independent manner, wherein said sustained release formulation comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient, and a pH-independent gel-forming polymer.

3. A sustained release formulation which comprises an active ingredient exhibiting a higher dissolution rate at pH 4 as compared with pH 1.2 or pH 6.8 and exerts a controlled release of said active ingredient in a pH-independent manner, wherein said sustained release formulation comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient, and a pH-independent gel-forming polymer, and contains substantially no pH-controlling agent other than said pH-independent gel-forming polymer.

4. The sustained release formulation according to any one of claims 1 to 3, wherein the pH-independent gel-forming polymer is at least one selected from the group consisting of hydroxypropylmethyl cellulose, polyethylene oxide, polyvinyl alcohol, hydroxypropyl cellulose and methyl cellulose.

5. The sustained release formulation according to claim 4, wherein the pH-independent gel-forming polymer is at least one selected from hydroxypropylmethyl cellulose, polyethylene oxide or polyvinyl alcohol.

6. The sustained release formulation according to any one of claims 1 to 3, wherein the content of the pH-independent gel-forming polymer is 10% by weight or more with respect to the total weight of the formulation.

7. The sustained release formulation according to any one of claims 1 to 3, wherein the content of the pH-independent gel-forming polymer is 10% by weight or more with respect to the active ingredient.

8. The sustained release formulation according to claim 4, wherein the pH-independent gel-forming polymer is hydroxypropylmethyl cellulose.

9. The sustained release formulation according to claim 8 which comprises hydroxypropylmethyl cellulose having a hydroxypropoxyl group content of 7 to 12% and a methoxy group content of 28 to 30%.

10. The sustained release formulation according to claim 8 which comprises hydroxypropylmethyl cellulose having a hydroxypropoxyl group content of 4 to 12% and a methoxy group content of 19 to 24%.

11. The sustained release formulation according to claim 8, wherein the content of hydroxypropylmethyl cellulose is 30% by weight or more with respect to the active ingredient.

12. The sustained release formulation according to claim 8, wherein the content of hydroxypropylmethyl cellulose is 40% by weight or more with respect to the active ingredient.

13. The sustained release formulation according to any one of claims 1 to 3 which further comprises a water insoluble material.

14. The sustained release formulation according to claim 13, wherein the water insoluble material is at least one selected from higher alcohols, waxes, hardened oils, ethyl cellulose and ethyl acrylate methyl methacrylate copolymers and aminoalkyl methacrylate copolymer RS.

15. The sustained release formulation according to claim 13, wherein the water insoluble material is at least one selected from higher alcohols and waxes.

16. A method for pH-independent controlled release of a sustained release formulation which comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient, wherein said method comprises dispersing ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride in a matrix of a pH-independent gel-forming polymer.

17. A pH adjusting agent for use in a sustained release formulation which comprise ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient and exerts a controlled release of said active ingredient in a pH-independent manner, wherein said agent consists of a pH-independent gel-forming polymer.

18. A method for preparing a sustained release formulation which comprises ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride as an active ingredient and exerts a controlled release of said active ingredient in a pH-independent manner, wherein said method comprises dispersing ethyl (-)-2-[4-[2-[[(1S,2R)-2-hydroxy-2-(4-hydroxyphenyl)-1-methylethyl]amino]ethyl]-2,5-dimethylphenoxy]acetate hydrochloride in a matrix of a pH-independent gel-forming polymer.
